# EUROPEAN PATENT APPLICATION

(11) **EP 4 101 416 A1**
(43) Date of publication of application: **14.12.2022**
(21) Application number: 20918089.2
(22) Date of filing: 05.02.2020
(51) Int. Cl.: A61C 1/07, A61B 17/32

(54) **ULTRASONIC PROBE AND TREATMENT SYSTEM**

(71) Applicant: Olympus Corporation, Hachioji-shi Tokyo 192-8507 (JP)
(72) Inventor: FUJISAKI, Ken, Hachioji-shi, Tokyo 192-8507 (JP)
(74) Representative: Schicker, Silvia
(86) International application number: PCT/JP2020/004271
(87) International publication number: WO 2021/156963

(57) **Abstract**

An ultrasound probe includes a treatment portion that is formed at a distal end of a transmission portion transmitting input vibration energy, the treatment portion being configured to vibrate with a predetermined amplitude to cut a treatment target object, in which the treatment portion includes a first striking face that faces the treatment target object, a second striking face that faces the treatment target object at a position away from the first striking face toward a proximal end side in an axial direction, and a first side surface that connects the first striking face and the second striking face in the axial direction and that has a predetermined length in the axial direction, in which when the predetermined length of the first side surface is h, 0 < h ≤ 200 [µm] is satisfied.

## Description

### Field

The present invention relates to an ultrasound probe and a treatment system that are used for an ultrasound treatment instrument.

### Background

There are known conventional ultrasound treatment instruments that include an ultrasound probe to form a bone hole in bone at a knee joint and the like.

Patent Literature 1 discloses an ultrasound treatment instrument that has steps in a rectangular distal end treatment portion provided on a distal end side in an axial direction of an ultrasound probe. In this ultrasound treatment instrument, the distal end treatment portion of the ultrasound probe is ultrasonically vibrated, cuts bone in a vibration direction, and forms a bone hole.

### Citation List

### Patent Literature

Patent Literature 1: WO 2019/008712 A

### Summary

### Technical Problem

However, the ultrasound treatment instrument disclosed in Patent Literature 1 is configured to cut bone in one direction, and performance of cutting in the vibration direction and cutting in a direction orthogonal to the vibration direction without interruption is not considered, thus cutting in a plurality of directions cannot be performed.

The present invention has been made in view of the above problem, and an object of the present invention is to provide an ultrasound probe and a treatment system that are configured to perform cutting in a vibration direction and cutting in a direction orthogonal to the vibration direction without interruption.

### Solution to Problem

In order to solve the above-described problem and achieve the object, an ultrasound probe according to the present invention includes a treatment portion that is formed at a distal end of a transmission portion transmitting input vibration energy, the treatment portion being configured to vibrate with a predetermined amplitude to cut a treatment target object. The treatment portion includes: a first striking face that faces the treatment target object; a second striking face that faces the treatment target object at a position away from the first striking face toward a proximal end side in an axial direction; and a first side surface that connects the first striking face and the second striking face in the axial direction and that has a predetermined length in the axial direction. When the predetermined length of the first side surface is h, 0 < h ≤ 200 [µm] is satisfied.

In the ultrasound probe according to the present invention, the predetermined length h of the first side surface satisfies 50 [µm] ≤ h ≤ 200 [µm].

In the ultrasound probe according to the present invention, the first side surface is recessed.

In the ultrasound probe according to the present invention, an angle between the first side surface and the second striking face is 90 degrees.

In the ultrasound probe according to the present invention, the treatment portion includes a second side surface that extends from an edge of the second striking face toward the proximal end side in the axial direction and that has a length different from the predetermined length of the first side surface in the axial direction.

In the ultrasound probe according to the present invention, the length of the second side surface in the axial direction is 200 [µm] or more.

A treatment system according to the present invention includes: the ultrasound probe according to the invention above; and a control device configured to control the vibration energy supplied to the ultrasound probe. The control device includes: a switching instruction portion configured to give an instruction to switch a drive mode; and an energy supply portion configured to supply the vibration energy generating ultrasound vibration having different amplitudes according to the instruction from the switching instruction portion.

An ultrasound probe according to the present invention includes a treatment portion that is formed at a distal end of a transmission portion transmitting input vibration energy, the treatment portion being configured to vibrate with a predetermined amplitude to cut a treatment target object. The treatment portion includes: a first striking face that faces the treatment target object; a second striking face that faces the treatment target object at a position away from the first striking face toward a proximal end side in an axial direction; and a first side surface that connects the first striking face and the second striking face in the axial direction and that has a predetermined length in the axial direction. The first side surface has a length that is equal to or less than an amplitude of vibration of the treatment portion caused by the input vibration energy.

### Advantageous Effects of Invention

In the ultrasound probe and the treatment system according to the present invention, the cutting in the vibration direction and the cutting in a direction orthogonal to the vibration direction can be effectively performed without interruption.

### Brief Description of Drawings

FIG. 1 is a diagram illustrating a system configuration example of an ultrasound treatment instrument according to an embodiment.
FIG. 2 is a diagram illustrating a configuration example of a treatment system including an ultrasound treatment instrument according to an embodiment.
FIG. 3A is a diagram illustrating an external shape of a distal end treatment portion of an ultrasound probe according to an embodiment, as viewed obliquely from above.
FIG. 3B is a side view of the distal end treatment portion of the ultrasound probe according to the embodiment, as viewed in a direction orthogonal to an axial direction.
FIG. 3C is a front view of the distal end treatment portion of the ultrasound probe according to the embodiment, as viewed from a distal end side in the axial direction.
FIG. 4 is a cross-sectional view of the distal end treatment portion taken along the line M-M in FIG. 3C.
FIG. 5A is a diagram illustrating a state in which the distal end treatment portion is being pushed to the maximum extent to the distal end side in the axial direction by ultrasound vibration upon vertically cutting a treatment target region.
FIG. 5B is a diagram illustrating a state in which the distal end treatment portion pulled back to the maximum extent to a proximal end side in the axial direction by the ultrasound vibration is separated from the treatment target region, from the state illustrated in FIG. 5A.
FIG. 5C is a diagram illustrating a state in which the distal end treatment portion is laterally moved from the state illustrated in FIG. 5B.
FIG. 5D is a diagram illustrating a state in which the distal end treatment portion pushed to the maximum extent to the distal end side in the axial direction by the ultrasound vibration performs further boring in the treatment target region, from the state illustrated in FIG. 5C.
FIG. 6A is a diagram illustrating movement of the distal end treatment portion upon making a long hole in the treatment target region.
FIG. 6B is a diagram illustrating the long hole made in the treatment target region as viewed in a direction indicated by an arrow P in FIG. 6A.
FIG. 7 is a diagram illustrating angles between side surfaces and striking faces in the distal end treatment portion, having an acute angle.
FIG. 8 is a diagram illustrating a side surface of the distal end treatment portion part of which is recessed in a direction orthogonal to the axial direction.
FIG. 9 is a diagram illustrating a case where axial lengths of a first side surface and a second side surface of the distal end treatment portion in the axial direction are different from each other.
FIG. 10A is a graph illustrating a first example of control of amplitude upon ultrasound vibration of the distal end treatment portion.
FIG. 10B is a graph illustrating a second example of control of amplitude upon ultrasound vibration of the distal end treatment portion.
FIG. 10C is a graph illustrating a third example of control of amplitude upon ultrasound vibration of the distal end treatment portion.
FIG. 11A is a side view of the distal end treatment portion of an ultrasound probe according to a second embodiment as viewed in a direction orthogonal to the axial direction.
FIG. 11B is a diagram illustrating a state in which the distal end treatment portion is moved forward and cut a treatment target region.
FIG. 11C is a diagram illustrating a state in which the distal end treatment portion is moved back and forth and cut the treatment target region.
FIG. 12A is a perspective view illustrating a distal end side of an ultrasound probe according to a third embodiment.
FIG. 12B is a diagram of a distal end treatment portion of the ultrasound probe according to the third embodiment as viewed from the distal end side in the axial direction.
FIG. 13A is a perspective view illustrating a distal end side of an ultrasound probe according to a fourth embodiment.
FIG. 13B is a diagram of a distal end treatment portion of the ultrasound probe according to the fourth embodiment as viewed from the distal end side in the axial direction.
FIG. 14A is a perspective view illustrating a distal end side of an ultrasound probe according to a fifth embodiment.
FIG. 14B is a diagram of a distal end treatment portion of the ultrasound probe according to the fifth embodiment as viewed from the distal end side in the axial direction.
FIG. 15A is a perspective view illustrating a distal end side of an ultrasound probe according to a sixth embodiment.
FIG. 15B is a diagram of a distal end treatment portion of the ultrasound probe according to the sixth embodiment as viewed from the distal end side in the axial direction.
FIG. 16 is a cross-sectional view of an ultrasound probe according to a seventh embodiment taken in the axial direction.

### Description of Embodiments

### (First Embodiment)

A treatment system according to a first embodiment of the present invention that includes an ultrasound treatment instrument including an ultrasound probe will be described below. It should be noted that the present invention is not limited to the present embodiment.

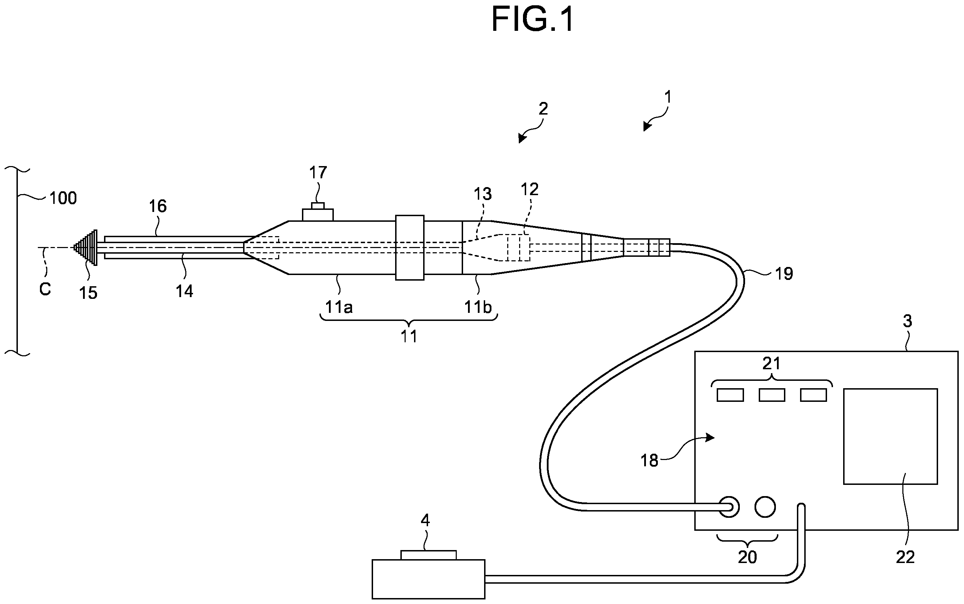

FIG. 1 is a diagram illustrating an ultrasound treatment instrument system 1 according to the first embodiment. The ultrasound treatment instrument system 1 according to the first embodiment mainly includes an ultrasound treatment instrument 2, a control device 3, and a foot switch 4 that gives an instruction for on/off of ultrasound vibration. The ultrasound treatment instrument 2 and the control device 3 are connected by a cable 19, and supply of drive power and communication of a control signal are performed. The control device 3 has a front surface 18 on which a plurality of connectors 20 for connection to the cable 19, various operation switches 21, and a display screen 22 that displays information necessary for treatment procedure are provided. Each of the operation switches 21 functions as, for example, a switching instruction portion that gives an instruction for switching drive modes of the ultrasound treatment instrument 2.

The ultrasound treatment instrument 2 includes a device body 11 and an ultrasound probe 14. The device body 11 has a cylindrical shape with a diameter for easy grasping, and includes a housing 11a through which the ultrasound probe 14 is arranged, and an ultrasound transducer unit 11b that is an ultrasound generation unit detachable from the housing 11a. The ultrasound transducer unit 11b internally stores an ultrasound generation unit 12 that includes an ultrasound vibration element such as a piezoelectric body, and a horn 13 that efficiently transmits an ultrasound wave. In a state where the ultrasound transducer unit 11b is mounted to the housing 11a, a proximal end side of the ultrasound probe 14 and a distal end side of the horn 13 are acoustically connected, and ultrasound vibration generated by the ultrasound generation unit 12 is transmitted to a distal end treatment portion 15 of the ultrasound probe 14 which is described later. The upper surface of the housing 11a is provided with an operation switch 17 that gives an instruction for on/off of ultrasound vibration according to an operator's finger operation. The foot switch 4 has a function of giving an instruction for on/off of ultrasound vibration according to an operator's foot operation.

The ultrasound probe 14 has an elongated rod-shaped shaft member (probe body) that transmits ultrasound vibration, and is made of a metal material such as a titanium alloy. The ultrasound probe 14 has a proximal end portion that is a vibration input portion to which vibration energy (ultrasound vibration) supplied from the ultrasound generation unit 12 is input. The ultrasound probe 14 has a distal end where the distal end treatment portion 15 is formed that cuts bone as a treatment target object by vibrating with a predetermined amplitude. The proximal end portion of the ultrasound probe 14 and the distal end treatment portion 15 are connected by the probe body that is a transmission portion transmitting the vibration energy (ultrasound vibration) input to the proximal end portion to the distal end treatment portion 15.

The ultrasound probe 14 is covered with a sheath 16 having an appropriate length from the housing 11a. The sheath 16 is not in close contact with the ultrasound probe 14, and has a slight gap to the ultrasound probe 14 so as not to attenuate the ultrasound vibration. The sheath 16 is fixed at a position of a node of the ultrasound vibration, on the distal end side of the housing 11a.

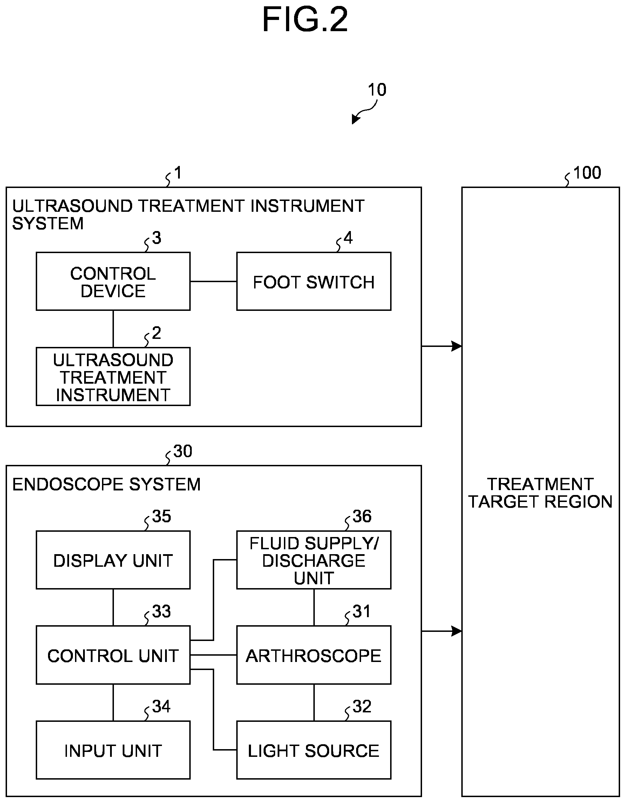

FIG. 2 is a block diagram illustrating a configuration of a treatment system 10 according to the first embodiment. The treatment system 10 according to the first embodiment performs a treatment procedure of forming a bone hole in a treatment target region 100 in the treatment target object such as femur, and includes the ultrasound treatment instrument system 1 and an endoscope system 30. As described above, the ultrasound treatment instrument system 1 includes the ultrasound treatment instrument 2, the control device 3, and the foot switch 4.

The endoscope system 30 includes an arthroscope 31 that includes a rigid endoscope being a type of endoscope, a light source 32 that emits visible illumination light, as a light source of illumination light, a control unit 33 that controls the entire endoscope system 30, an input unit 34 such as a keyboard or a touch panel, a display unit 35 that displays treatment information including a captured treatment situation, and a fluid supply/discharge unit 36 that supplies, discharges, or perfuses a saline solution to and from the treatment target region 100 and the periphery thereof. In the present embodiment, the fluid supply/discharge unit 36 is configured to supply and discharge the saline solution to and from the treatment target region 100 and the periphery thereof through the arthroscope 31, but may be configured to supply and discharge perfusate containing saline solution or the like, from the ultrasound treatment instrument 2.

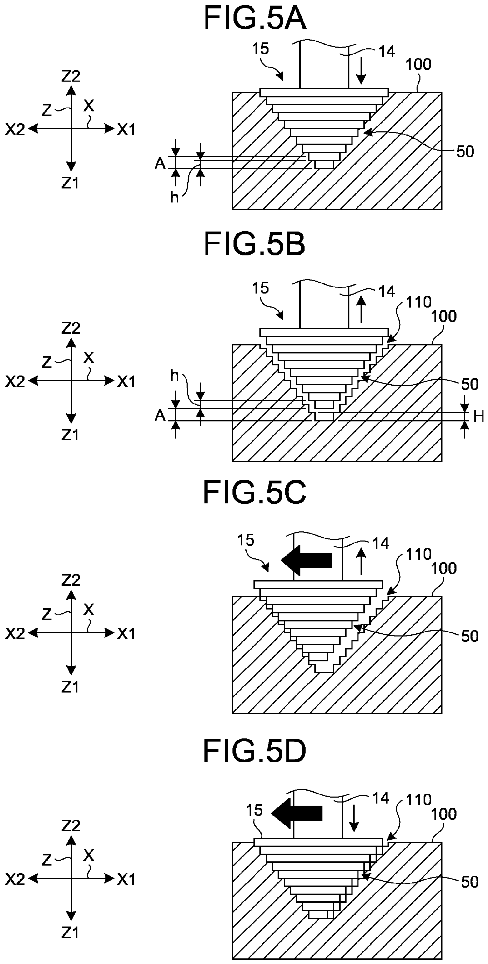

Here, in a case where a cell structure of cancellous bone is crushed to form a bone hole 110 (see FIG. 5) in the treatment target region 100 by using the ultrasound treatment instrument 2, the amplitude of ultrasound vibration that is larger than a cell width of the cell structure of the cancellous bone does not crush the cell structure but often breaks the cell structure. Therefore, in order to form the bone hole 110 in the treatment target region 100 by using the ultrasound treatment instrument 2, it is desirable to set the amplitude of the ultrasound vibration to 200 [µm] or less.

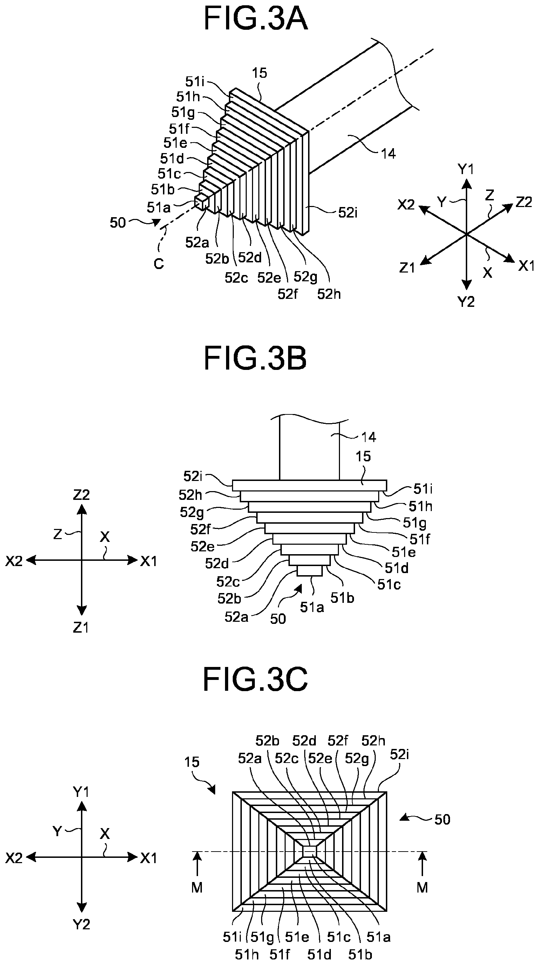

Next, a structure of the distal end treatment portion 15 of the ultrasound probe 14 will be described. FIG. 3A is a diagram illustrating an external shape of the distal end treatment portion 15 of the ultrasound probe 14 according to the embodiment, as viewed obliquely from above. Note that in each of FIGS. 3A, 3B, and 3C, an arrow Z indicates an axial direction, Z1 denotes the distal end side, and Z2 denotes the proximal end side. Furthermore, in each of FIGS. 3A, 3B, and 3C, an arrow X indicates a first orthogonal direction orthogonal to the axial direction Z, X1 denotes one side, and X2 denotes the other side. In FIGS. 3A, 3B, and 3C, an arrow Y indicates a second orthogonal direction orthogonal to the axial direction Z and the first orthogonal direction X, Y1 denotes one side, and Y2 denotes the other side.

FIG. 3B is a side view of the distal end treatment portion 15 of the ultrasound probe 14 according to the embodiment, as viewed in a direction orthogonal to the axial direction Z. FIG. 3C is a front view of the distal end treatment portion 15 of the ultrasound probe 14 according to the embodiment, as viewed from the distal end side Z1 in the axial direction Z.

As illustrated in FIGS. 3A, 3B, and 3C, the distal end treatment portion 15 of the present embodiment has a substantially quadrangular pyramid shape that has a bottom surface connected to the probe body of the ultrasound probe 14, and four side surfaces each provided with stair-like steps.

The distal end treatment portion 15 according to the first embodiment includes a striking face portion 50 protruding toward the distal end side Z1 in the axial direction Z. The striking face portion 50 includes a striking face 51a to a striking face 51i that face the distal end side Z1 in the axial direction Z, and a side surface 52a to a side surface 52h that extend from the edges of the striking face 51a to the striking face 51i to the proximal end side Z2 in the axial direction Z. Then, the striking faces 51a to 51i and the side surfaces 52a to 52h form stair-like steps on the outer peripheral surface of the striking face portion 50. In addition, the striking face 51a is a distal end surface of the distal end treatment portion 15 and is formed into a square flat surface.

Note that in the following description, the striking faces 51a to 51i which are not particularly distinguished are also simply referred to as striking faces 51, in some cases. Furthermore, the side surfaces 52a to 52h which are not particularly distinguished are also simply referred to as the side surfaces 52, in some cases.

Here, in the striking face portion 50, striking faces 51 arranged adjacently in the axial direction Z are connected by a side surface 52, and a striking face 51 positioned on the distal end side Z1 in the axial direction Z is defined as a first striking face, and a striking face 51 positioned on the proximal end side Z2 in the axial direction Z is defined as a second striking face. For example, in the striking face portion 50, as for the striking face 51a and the striking face 51b which are arranged adjacently in the axial direction Z, the striking face 51a positioned on the distal end side Z1 in the axial direction Z is the first striking face, and the striking face 51b positioned on the proximal end side Z2 in the axial direction Z is the second striking face. In the striking face portion 50, an angle between a side surface 52 and the second striking face is 90 degrees. In addition, in the striking face portion 50, a side surface 52 connecting the first striking face and the second striking face is defined as a first side surface. For example, the side surface 52a connecting the striking face 51a and the striking face 51b is the first side surface.

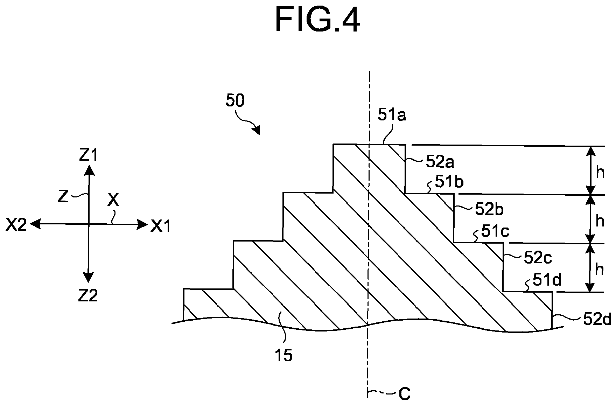

FIG. 4 is a cross-sectional view of the distal end treatment portion taken along the line M-M in FIG. 3C. Note that FIG. 4 illustrates only a part of the distal end side Z1 in the axial direction Z in the distal end treatment portion 15. Furthermore, in FIG. 4, "h" represents a length of a side surface 52 of the striking face portion 50 in the distal end treatment portion 15 in the axial direction Z, in other words, a height of a step between striking faces arranged adjacently in the axial direction Z.

In the first embodiment, the length h of a side surface 52 connecting striking faces arranged adjacently in the axial direction Z of the striking face portion 50 in the distal end treatment portion 15 in the axial direction Z satisfies 0 < h ≤ 200 [µm], more preferably satisfies 50 [µm] ≤ h ≤ 200 [µm].

The distal end treatment portion 15 has a function to be vibrated in the axial direction Z by ultrasound vibration, repeatedly strike the striking faces 51a to 51i of the striking face portion 50 against the treatment target region 100, and cut the treatment target region 100.

As illustrated in FIG. 5A, when the treatment target region 100 is cut by the distal end treatment portion 15, the inner surface of the bone hole 110 is cut into stair-like steps in conformity with the outer peripheral surface of the striking face portion 50 by the striking faces 51 of the striking face portion 50. Therefore, a step H formed on the inner surface of the bone hole 110 cut into the stair-like steps is equal to the height of a step (the length h of the side surface 52 in the axial direction Z) formed on the outer peripheral surface of the striking face portion 50. Then, as illustrated in FIG. 5A, in a state where the distal end treatment portion 15 is being pushed to the maximum extent to the distal end side Z1 in the axial direction Z by the ultrasound vibration, the inner surface of the bone hole 110 and the distal end treatment portion 15 are in contact with each other.

Note that "A" in FIG. 5A represents the amplitude of the vibration of the distal end treatment portion 15 vibrated in the axial direction Z by the ultrasound vibration. In addition, the amplitude A corresponds to a distance in the axial direction Z between a distal end position when the distal end treatment portion 15 is pushed to the maximum extent to the distal end side Z1 in the axial direction Z and a position pulled back to the maximum extent to the proximal end side Z2 in the axial direction Z by the ultrasound vibration. In the first embodiment, the height of a step formed on the outer peripheral surface of the striking face portion 50 in the distal end treatment portion 15 (the length of a side surface 52 in the axial direction Z) is set to be equal to or less than the amplitude A. For example, when the amplitude A of the vibration of the distal end treatment portion 15 in the axial direction Z is 30 [µm], the length h of the side surface 52 in the axial direction Z is set to 15 [µm]. Furthermore, when the amplitude A is set to 60 [µm], the length h is set to 40 [µm]. Furthermore, when the amplitude A is set to 120 [µm], the amplitude A of the vibration of the distal end treatment portion 15 in the axial direction Z and the length h of the side surface 52 in the axial direction Z are preferably designed according to cutting, such as by setting the length h to a length of 90 [µm]. In the following embodiments, the length h of the side surface 52 relative to the amplitude A can be determined with a similar concept.

As illustrated in FIG. 5B, in a state where the distal end treatment portion 15 is pulled back to the maximum extent to the proximal end side Z2 in the axial direction Z by the ultrasound vibration, from the state illustrated in FIG. 5A, the striking faces 51 and the side surfaces 52 of the striking face portion 50 make no contact with the inner surface of the bone hole 110. At this time, between the inner surface of the bone hole 110 and the distal end treatment portion 15, a gap is formed that enables movement of the striking faces 51 of the striking face portion 50 over the steps formed on the inner surface of the bone hole 110 and movement of the distal end treatment portion 15 in a direction orthogonal to the axial direction Z. Therefore, in the present embodiment, as illustrated in FIG. 5C, while the distal end treatment portion 15 is pulled back to the maximum extent to the proximal end side Z2 in the axial direction Z by the ultrasound vibration, the distal end treatment portion 15 is movable in the bone hole 110 in the direction orthogonal to the axial direction Z. Then, as illustrated in FIG. 5D, the distal end treatment portion 15 is pushed to the distal end side Z1 in the axial direction Z by the ultrasound vibration, and whereby the inner surface of the bone hole 110 is further cut by the striking faces 51 of the striking face portion 50 in the distal end treatment portion 15, from the state of FIG. 5C.

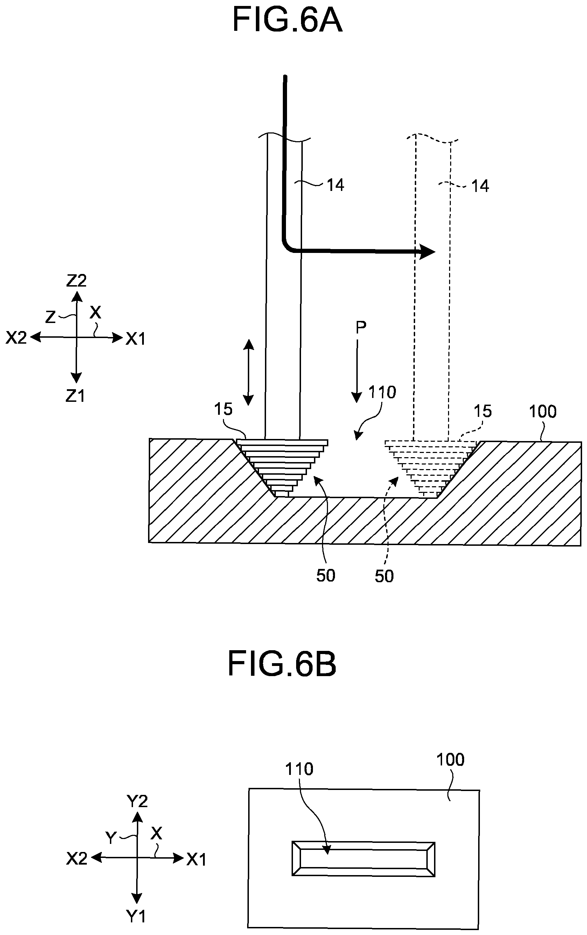

As described above, in the present embodiment, as illustrated in FIG. 6A, vertical cutting that is cutting of the treatment target region 100 in the axial direction Z (vibration direction) by the distal end treatment portion 15 and horizontal cutting that is cutting of the treatment target region 100 in a direction orthogonal to the axial direction Z (vibration direction) by the distal end treatment portion 15 can be performed without interruption. This configuration makes it possible to improve the operability of the ultrasound treatment instrument 2 by the operator when the bone hole 110 of elongated shape as illustrated in FIG. 6B is formed in the treatment target region 100.

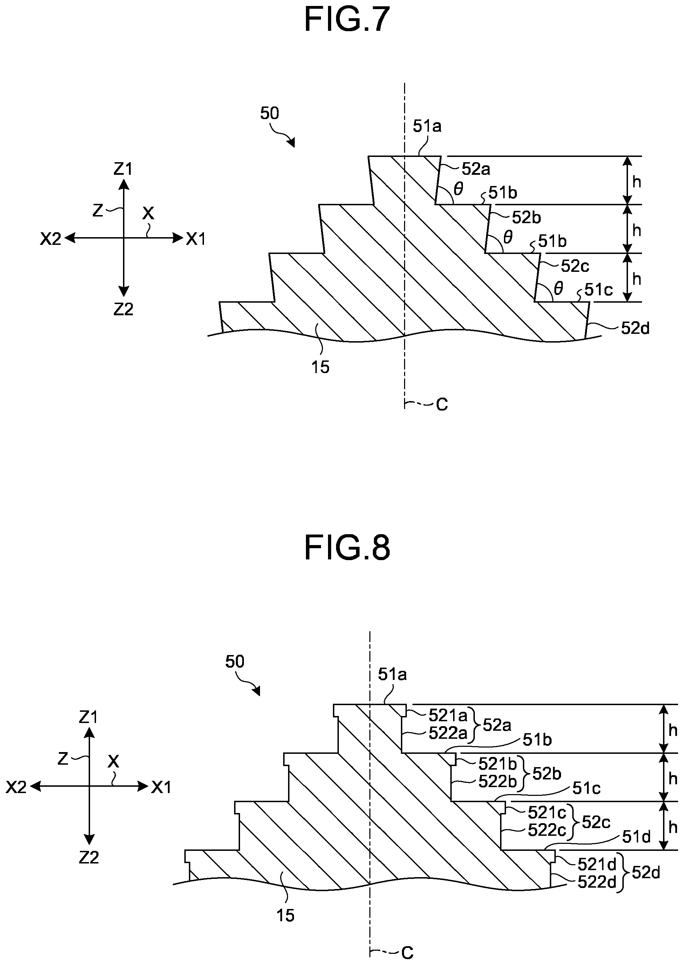

Note that in the distal end treatment portion 15 of the first embodiment, the angle between the side surface 52 and the second striking face in the striking face portion 50 is not limited to 90 degrees as illustrated in FIG. 4. For example, as illustrated in FIG. 7, an angle θ between the side surface 52 and the second striking face may be smaller than 90 degrees. In other words, each side surface 52 may extend from the distal end side Z1 (first striking face side) toward the proximal end side Z2 (second striking face side) so as to be inclined downward to the axial direction Z.

In addition, in the distal end treatment portion 15 of the first embodiment, as illustrated in FIG. 8, part of each side surface 52 of the striking face portion 50 may be recessed in a direction orthogonal to the axial direction Z. For example, in the striking face portion 50, part of the side surface 52a connecting the striking face 51a as the first striking face and the striking face 51b as the second striking face in the axial direction Z may be recessed in a direction orthogonal to the axial direction Z. Specifically, the side surface 52a may be divided in the axial direction Z into a side surface portion 521a connected to the striking face 51a being the first striking face and a side surface portion 522a connected to the striking face 51b being the second striking face so that the side surface portion 522a is provided inner than the side surface portion 521a in the first orthogonal direction X. Furthermore, also in the side surfaces 52b to 52d, side surface portions 522b to 522d may be provided inward from side surface portions 521b to 521d in the first orthogonal direction X.

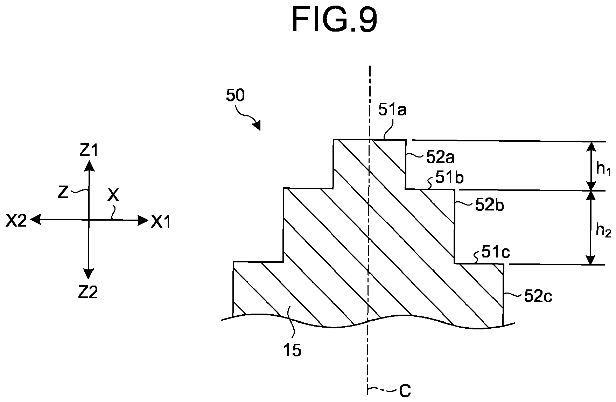

In addition, in the distal end treatment portion 15 of the first embodiment, as illustrated in FIG. 9, the lengths in the axial direction Z of side surfaces 52 of the striking face portion 50 arranged adjacently in the axial direction Z may be different from each other. For example, a length h₁ in the axial direction Z of the side surface 52a being the first side surface connecting the striking face 51a and the striking face 51b and a length h₂ in the axial direction Z of the side surface 52b being a second side surface connecting the striking face 51b and the striking face 51c may satisfy a relationship of h₁ < h₂. At this time, for example, the lengths h₁ and h₂ can satisfy 0 < h₁ ≤ 200 [µm], and h₂ > 200 [µm].

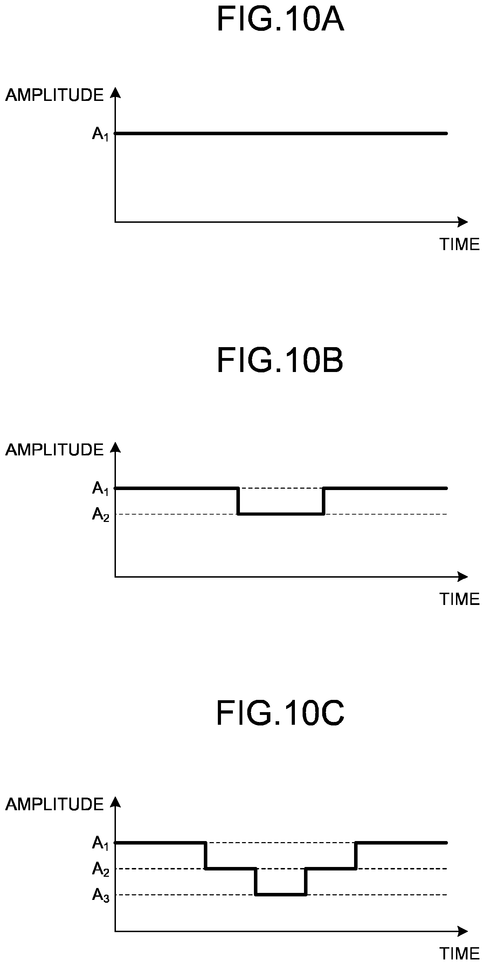

FIG. 10A is a graph illustrating a first example of control of the amplitude upon ultrasound vibration of the distal end treatment portion 15. In FIG. 10A, when the bone hole 110 is formed in the treatment target region 100 by the ultrasound treatment instrument 2, the distal end treatment portion 15 is ultrasonically vibrated only with an amplitude A₁ larger than the length h (height of the step) in the axial direction Z of the side surface 52 of the striking face portion 50 in the distal end treatment portion 15.

FIG. 10B is a graph illustrating a second example of the control of amplitude upon ultrasound vibration of the distal end treatment portion. FIG. 10B illustrates ultrasound vibration of the distal end treatment portion 15 performed by selectively switching the amplitude A₁ larger than the length h (height of the step) in the axial direction Z of the side surface 52 of the striking face portion 50 in the distal end treatment portion 15 and an amplitude A₂ smaller than the length h (height of the step), when the bone hole 110 is formed in the treatment target region 100 by the ultrasound treatment instrument 2. In FIG. 10B, a drive mode for ultrasound vibration with the amplitude A1 and a drive mode for ultrasound vibration with the amplitude A2 are switched by operating the operation switches 21 of the control device 3 by the operator.

When the bone hole 110 is formed in the treatment target region 100 by the ultrasound treatment instrument 2, with the distal end treatment portion 15 ultrasonically vibrated with the amplitude A₁, the striking faces 51 are movable over the steps formed on the inner surface of the bone hole 110. Accordingly, the distal end treatment portion 15 can be moved in a direction orthogonal to the axial direction Z, such as the first orthogonal direction X or the second orthogonal direction Y, in the bone hole 110 to perform the horizontal cutting. In addition, switching the amplitude of the ultrasound vibration from the amplitude A₁ to the amplitude A₂ prevents the striking faces 51 from moving over the steps formed on the inner surface of the bone hole 110, preventing the horizontal cutting by moving the distal end treatment portion 15 in a direction orthogonal to the axial direction Z, such as the first orthogonal direction X or the second orthogonal direction Y, in the bone hole 110. Meanwhile, the ultrasound vibration of the distal end treatment portion 15 with the amplitude A₂ improves the straight movement in the vertical cutting, compared with the ultrasound vibration of the distal end treatment portion 15 with the amplitude A₁, improving processability in the vertical cutting.

Therefore, in the ultrasound treatment instrument 2 according to the first embodiment, for example, the bone hole 110 can be formed in the treatment target region 100 as follows. First, the horizontal cutting is performed by the distal end treatment portion 15 ultrasonically vibrated with the amplitude A₁, as illustrated in FIG. 6A to form the bone hole 110 of elongated shape having a constant depth in the treatment target region 100. Then, the amplitude of the ultrasound vibration is switched from the amplitude A₁ to the amplitude A₂, the distal end treatment portion 15 is moved in the axial direction Z, and the bone hole 110 is bored deeper to a desired depth by the vertical cutting. Then, after boring the hole to the desired depth, the amplitude of the ultrasound vibration is switched from the amplitude A₂ to the amplitude A₁, and the horizontal cutting is performed by moving the distal end treatment portion 15 in a longitudinal direction in the bone hole 110 so that the bone hole 110 may have the desired depth in the longitudinal direction of the bone hole 110. This configuration makes it possible to reduce a time required to bore the bone hole 110 deeper in the treatment target region 100 and further reduce a time required for treatment procedure, compared with ultrasound vibration of the distal end treatment portion 15 only with the amplitude A₁ larger than the length h (height of the step).

In addition, the amplitude of the ultrasound vibration is not limited to two levels of the amplitude A₁ and the amplitude A₂, upon selectively switching the amplitude A₁ larger than the length h (height of the step) in the axial direction of the side surface 52 of the striking face portion 50 in the distal end treatment portion 15 and the amplitude A₂ smaller than the length h (height of the step), when the bone hole 110 is formed in the treatment target region 100 by the ultrasound treatment instrument 2. For example, as illustrated in FIG. 10C, the amplitude of the ultrasound vibration may be selectively switched between three levels of the amplitude A₁, the amplitude A₂, and an amplitude A₃ that is smaller than the amplitude A₂. At this time, switching of the amplitudes of the ultrasound vibration may be performed, for example, by switching the drive modes for ultrasound vibration with the amplitudes A₁, A₂, and A₃ through the operation of the operation switches 21 of the control device 3 by the operator, for stepwisely switching the amplitude A₁, the amplitude A₂, and the amplitude A₃, or for switching between the amplitude A₁ and the amplitude A₂ or between the amplitude A₁ and the amplitude A₃.

### (Second Embodiment)

The treatment system according to a second embodiment of the present invention that includes the ultrasound treatment instrument including the ultrasound probe will be described below. Note that, in the second embodiment, description of portions common to those in the first embodiment will be omitted as appropriate.

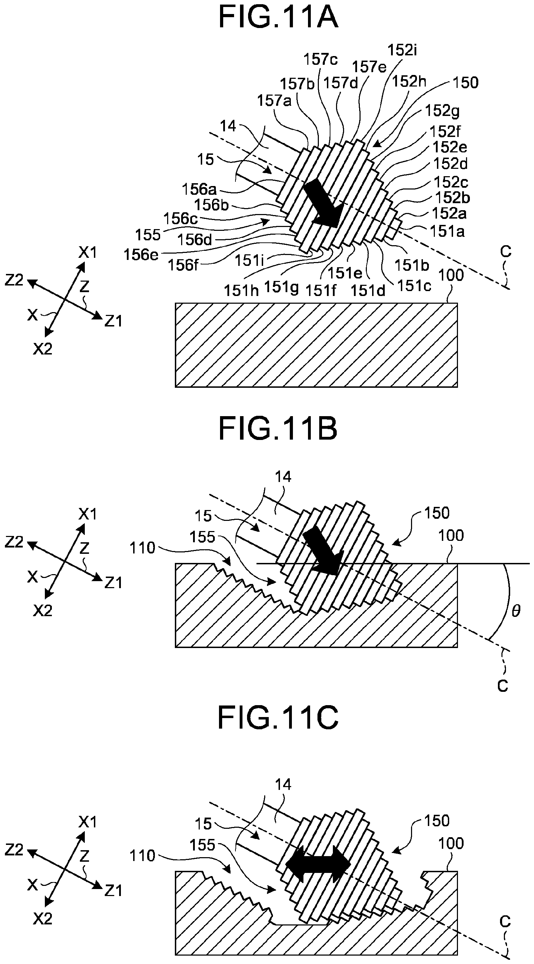

FIG. 11A is a side view of the distal end treatment portion 15 of the ultrasound probe 14 according to the second embodiment as viewed in a direction orthogonal to the axial direction Z. FIG. 11B is a diagram illustrating a state in which the distal end treatment portion 15 is moved forward and cut the treatment target region 100. FIG. 11C is a diagram illustrating a state in which the distal end treatment portion 15 is moved back and forth and cut the treatment target region 100.

The distal end treatment portion 15 according to the second embodiment includes a distal-end-side striking face portion 150 protruding toward the distal end side Z1 in the axial direction Z and a proximal-end-side striking face portion 155 protruding toward the proximal end side Z2 in the axial direction Z, and the distal-end-side striking face portion 150 and the proximal-end-side striking face portion 155 are provided to be connected in the axial direction Z.

The distal-end-side striking face portion 150 includes a striking face 151a to a striking face 151i that face the distal end side Z1 in the axial direction Z, and a side surface 152a to a side surface 152i that face in a direction orthogonal to the axial direction Z and each of which connects two striking faces arranged adjacently in the axial direction Z. Then, the striking faces 151a to 151i and the side surfaces 152a to 152i form stair-like steps on the outer peripheral surface of the distal-end-side striking face portion 150. In addition, the striking face 151a is a distal end surface of the distal end treatment portion 15 and is formed into a square flat surface.

Note that in the following description, the striking faces 151a to 151i which are not particularly distinguished are also simply referred to as striking faces 151, in some cases. Furthermore, the side surfaces 152a to 152i which are not particularly distinguished are also simply referred to as the side surfaces 152, in some cases.

Here, in the distal-end-side striking face portion 150, of striking faces arranged adjacently in the axial direction Z, a striking face 151 positioned on the distal end side Z1 in the axial direction Z is defined as the first striking face, and a striking face 151 positioned on the proximal end side Z2 in the axial direction Z is defined as the second striking face. For example, in the distal-end-side striking face portion 150, as for the striking face 151a and the striking face 151b which are arranged adjacently in the axial direction Z, the striking face 151a positioned on the distal end side Z1 in the axial direction Z is the first striking face, and the striking face 151b positioned on the proximal end side Z2 in the axial direction Z is the second striking face. Furthermore, in the distal-end-side striking face portion 150, as for the striking face 151b and the striking face 151c which are arranged adjacently in the axial direction Z, the striking face 151b positioned on the distal end side Z1 in the axial direction Z is the first striking face, and the striking face 151c positioned on the proximal end side Z2 in the axial direction Z is the second striking face. Then, in the distal-end-side striking face portion 150, an angle between a side surface 152 and the second striking face is 90 degrees. Note that the angle may be smaller than 90 degrees. Furthermore, part of the side surface 152 may be recessed in a direction orthogonal to the axial direction Z.

The proximal-end-side striking face portion 155 includes a striking face 156a to a striking face 156f that face the proximal end side Z2 in the axial direction Z, and a side surface 157a to a side surface 157e that face in a direction orthogonal to the axial direction Z and each of which connects two striking faces arranged adjacently in the axial direction Z. Then, the striking faces 156a to 156f and the side surfaces 157a to 157e form stair-like steps on the outer peripheral surface of the proximal-end-side striking face portion 155. In addition, the striking face 156a is a proximal end surface of the distal end treatment portion 15 and is formed into a square flat surface. The probe body of the ultrasound probe 14 is connected to the striking face 156a.

Note that in the following description, the striking faces 156a to 156f which are not particularly distinguished are also simply referred to as striking faces 156, in some cases. Furthermore, the side surfaces 157a to 157e which are not particularly distinguished are also simply referred to as the side surfaces 157, in some cases.

Here, in the proximal-end-side striking face portion 155, of striking faces arranged adjacently in the axial direction Z, a striking face 156 positioned on the proximal end side Z2 in the axial direction Z is defined as the first striking face, and a striking face 156 positioned on the distal end side Z1 in the axial direction Z is defined as the second striking face. For example, in the proximal-end-side striking face portion 155, as for the striking face 156a and the striking face 156b which are arranged adjacently in the axial direction Z, the striking face 156a positioned on the proximal end side Z2 in the axial direction Z is the first striking face, and the striking face 156b positioned on the distal end side Z1 in the axial direction Z is the second striking face. In addition, in the proximal-end-side striking face portion 155, as for the striking face 156b and the striking face 156c which are arranged adjacently in the axial direction Z, the striking face 156b positioned on the proximal end side Z2 in the axial direction Z is the first striking face, and the striking face 156c positioned on the distal end side Z1 in the axial direction Z is the second striking face. Then, in the proximal-end-side striking face portion 155, an angle between a side surfaces 157 and the second striking face is 90 degrees. Note that the angle may be smaller than 90 degrees. Furthermore, part of the side surfaces 157 may be recessed in a direction orthogonal to the axial direction Z.

In addition, the striking face 151i of the distal-end-side striking face portion 150 and the striking face 156f of the proximal-end-side striking face portion 155 are connected by the side surface 152i extending in the axial direction Z.

In the distal end treatment portion 15 of the second embodiment, a height of a step formed on the outer peripheral surface of the distal-end-side striking face portion 150 (length of a side surface 152 in the axial direction Z) and a height of a step formed on the outer peripheral surface of the proximal-end-side striking face portion 155 (length of a side surface 157 in the axial direction Z) are set to be equal to or less than the amplitude A of ultrasound vibration of the distal end treatment portion 15.

Therefore, when the bone hole 110 is formed in the treatment target region 100 by the ultrasound treatment instrument 2, with the distal end treatment portion 15 ultrasonically vibrated with the amplitude A, the striking faces 151 of the distal-end-side striking face portion 150 or the striking faces 156 of the proximal-end-side striking face portion 155 are movable over the steps formed on the inner surface of the bone hole 110. Accordingly, the distal end treatment portion 15 can be moved in a direction orthogonal to the axial direction Z, such as the first orthogonal direction X or the second orthogonal direction Y, in the bone hole 110 to perform the horizontal cutting. This configuration makes it possible to improve the operability of the ultrasound treatment instrument 2 by the operator when the bone hole 110 of elongated shape is formed in the treatment target region 100.

In the distal end treatment portion 15 according to the second embodiment, the distal end treatment portion 15 pushed toward the distal end side Z1 in the axial direction Z by the ultrasound vibration strikes the striking faces 151 of the distal-end-side striking face portion 150 against the treatment target region 100, and the treatment target region 100 can be cut. In addition, the distal end treatment portion 15 pulled back to the proximal end side Z2 in the axial direction Z by the ultrasound vibration strikes the striking faces 156 of the proximal-end-side striking face portion 155 against the treatment target region 100, and the treatment target region 100 can be cut.

In addition, when the bone hole 110 is formed in the treatment target region 100 by using the ultrasound treatment instrument 2 according to the second embodiment, for example, the treatment target region 100 can be cut, with the axis of the distal end treatment portion 15 inclined relative to the surface of the treatment target region 100, as illustrated in FIG. 11A. In other words, as illustrated in FIG. 11B, when the distal end treatment portion 15 ultrasonically vibrated is moved forward in the bone hole 110, the treatment target region 100 can be cut by the distal-end-side striking face portion 150. Note that at this time, the distal end treatment portion 15 is pressed not only in the axial direction Z but also in a direction orthogonal to the axial direction Z.

In addition, in the ultrasound treatment instrument 2 according to the second embodiment, as illustrated in FIG. 11C, when the distal end treatment portion 15 ultrasonically vibrated is moved backward in the bone hole 110, the treatment target region 100 can be cut by the distal-end-side striking face portion 150 and the proximal-end-side striking face portion 155. This configuration makes it possible to improve the operability of the ultrasound treatment instrument 2 by the operator when the bone hole 110 of elongated shape is formed in the treatment target region 100.

### (Third Embodiment)

The treatment system according to a third embodiment of the present invention that includes the ultrasound treatment instrument including the ultrasound probe will be described below. Note that, in the third embodiment, description of portions common to those in the first embodiment will be omitted as appropriate.

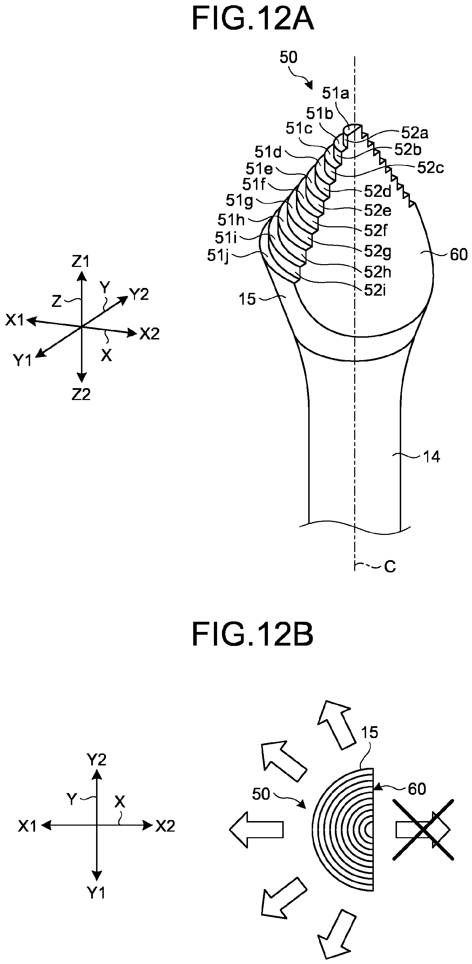

FIG. 12A is a perspective view illustrating the distal end side Z1 of the ultrasound probe 14 according to the third embodiment. FIG. 12B is a diagram of the distal end treatment portion 15 of the ultrasound probe 14 according to the third embodiment as viewed from the distal end side Z1 in the axial direction Z.

As illustrated in FIG. 12A, the ultrasound probe 14 of the third embodiment includes the striking face portion 50 provided around a half of an axis C of the distal end treatment portion 15 and a side surface portion 60 provided around the other half of the axis of the distal end treatment portion 15.

In the striking face portion 50, a striking face 51a to a striking face 51j and a side surface 52a to a side surface 52h form a stair-like steps and a side surface connecting the first striking face and the second striking face that are arranged adjacently in the axial direction Z has a length (height of a step) that is equal to or less than the amplitude A of the ultrasound vibration. Therefore, the distal end treatment portion 15 ultrasonically vibrated with the amplitude A and being cutting the bone hole 110 is movable, in a direction orthogonal to the axial direction Z, to a side on which the striking face portion 50 is provided, such as the one side X1 in the first orthogonal direction X, as indicated by arrows in FIG. 12B. On the other hand, the distal end treatment portion 15 ultrasonically vibrated with the amplitude A and being cutting the bone hole 110 cannot move to the other side X2 in the first orthogonal direction X on which the side surface portion 60 is provided, and a moving direction of the distal end treatment portion 15 is limited. This configuration makes it possible to suppress movement of the distal end treatment portion 15 ultrasonically vibrated with the amplitude A in an unintended direction during cutting in the bone hole 110.

In addition, in the distal end treatment portion 15 of the third embodiment, the side surface portion 60 provided on the other side X2 in the first orthogonal direction X is moved along the inner surface of the bone hole 110, and thus, straight movement in cutting in the vibration direction can be improved.

### (Fourth Embodiment)

The treatment system according to a fourth embodiment of the present invention that includes the ultrasound treatment instrument including the ultrasound probe will be described below. Note that, in the fourth embodiment, description of portions common to those in the first embodiment will be omitted as appropriate.

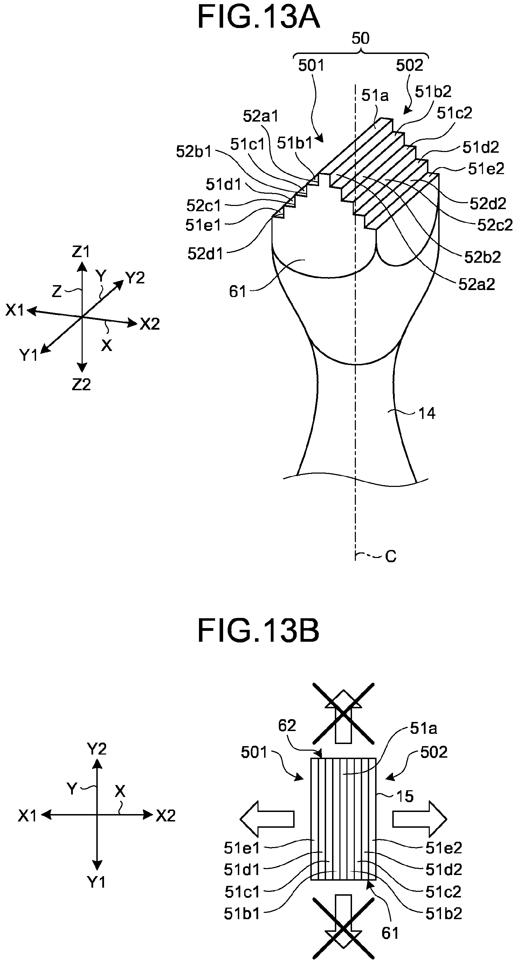

FIG. 13A is a perspective view illustrating the distal end side Z1 of the ultrasound probe 14 according to the fourth embodiment. FIG. 13B is a diagram of the distal end treatment portion 15 of the ultrasound probe 14 according to the fourth embodiment as viewed from the distal end side Z1 in the axial direction Z.

As illustrated in FIGS. 13A and 13B, the distal end treatment portion 15 of the fourth embodiment is provided with the striking face portion 50 including a first striking face portion 501 and a second striking face portion 502 that have steps symmetrical about the axis C in the first orthogonal direction X. In addition, the distal end treatment portion 15 of the fourth embodiment includes side surface portions 61 and 62 formed into flat surfaces, on the one side Y1 and the other side Y2 in the second orthogonal direction Y.

In the first striking face portion 501, a striking face 51a to a striking face 51e1 and a side surface 52a1 to a side surface 52d1 form a stair-like steps and a side surface connecting the first striking face and the second striking face that are arranged adjacently in the axial direction Z has a length (height of a step) that is equal to or less than the amplitude A of the ultrasound vibration. In addition, in the second striking face portion 502, a striking face 51a to a striking face 51e2 and a side surface 52a2 to a side surface 52d2 form a stair-like steps and a side surface connecting the first striking face and the second striking face that are arranged adjacently in the axial direction Z has a length (height of a step) that is equal to or less than the amplitude A of the ultrasound vibration. Therefore, the distal end treatment portion 15 ultrasonically vibrated with the amplitude A and being cutting the bone hole 110 is movable to the one side X1 in the first orthogonal direction X on which the first striking face portion 501 is provided and further movable to the other side X2 in the first orthogonal direction X on which the second striking face portion 502 is provided, as indicated by arrows in FIG. 13B. On the other hand, the distal end treatment portion 15 ultrasonically vibrated with the amplitude A and being cutting the bone hole 110 cannot move to the one side Y1 and the other side Y2 in the second orthogonal direction Y on which the side surface portions 61 and 62 are provided, and the moving direction of the distal end treatment portion 15 is limited. This configuration makes it possible to suppress movement of the distal end treatment portion 15 ultrasonically vibrated with the amplitude A in an unintended direction during cutting in the bone hole 110.

In addition, in the distal end treatment portion 15 of the fourth embodiment, at least one of the side surface portions 61 and 62 provided on the one side Y1 and the other side Y2 in the second orthogonal direction Y is moved along the inner surface of the bone hole 110, and thus, straight movement in cutting in the vibration direction can be improved.

### (Fifth Embodiment)

The treatment system according to a fifth embodiment of the present invention that includes the ultrasound treatment instrument including the ultrasound probe will be described below. Note that, in the fifth embodiment, description of portions common to those in the first embodiment will be omitted as appropriate.

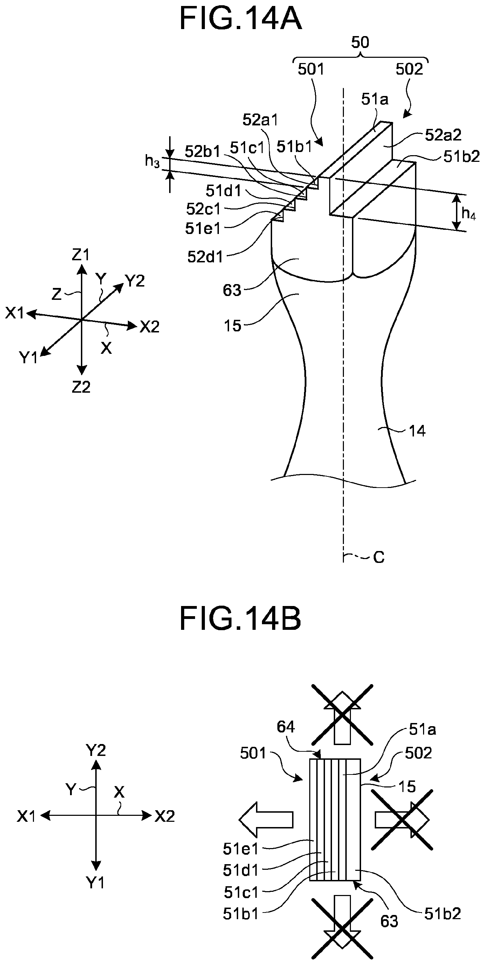

FIG. 14A is a perspective view illustrating the distal end side Z1 of the ultrasound probe 14 according to the fifth embodiment. FIG. 14B is a diagram of the distal end treatment portion 15 of the ultrasound probe 14 according to the fifth embodiment as viewed from the distal end side Z1 in the axial direction Z.

As illustrated in FIGS. 14A and 14B, in the distal end treatment portion 15 of the fifth embodiment includes, on both sides of the axis C, the first striking face portion 501 that is provided on the one side X1 in the first orthogonal direction X and the second striking face portion 502 that is provided on the other side X2 in the first orthogonal direction X. In addition, the distal end treatment portion 15 of the fifth embodiment includes side surface portions 63 and 64 formed into flat surfaces, on the one side Y1 and the other side Y2 in the second orthogonal direction Y, on both sides of the axis C.

The first striking face portion 501 and the second striking face portion 502 are different in length of a side surface (height of a step) connecting the first striking face and the second striking face that are arranged adjacently in the axial direction Z. In other words, a length h₃ of the side surface 52a1 connecting the striking faces 51a and 51b1 in the first striking face portion 501 satisfies 0 < h₃ ≤ 200 [µm] and is equal to or less than the amplitude A of the ultrasound vibration. Meanwhile, a length h₄ of the side surfaces 52a2 connecting the striking faces 51a and 51b2 in the second striking face portion 502 satisfies h₄ > 200 [µm] and is larger than the amplitude A of the ultrasound vibration.

Therefore, the distal end treatment portion 15 ultrasonically vibrated with the amplitude A and being cutting the bone hole 110 is movable to the one side X1 in the first orthogonal direction X on which the first striking face portion 501 is provided, as indicated by an arrow in FIG. 14B. On the other hand, the distal end treatment portion 15 ultrasonically vibrated with the amplitude A and being cutting the bone hole 110 cannot move to the other side X2 in the first orthogonal direction X on which the second striking face portion 502 is provided and the one side Y1 and the other side Y2 in the second orthogonal direction Y on which the side surface portions 63 and 64 are provided, and the moving direction of the distal end treatment portion 15 is limited. This configuration makes it possible to suppress movement of the distal end treatment portion 15 ultrasonically vibrated with the amplitude A in an unintended direction during cutting in the bone hole 110.

In addition, in the distal end treatment portion 15 of the fifth embodiment, at least one of the side surface portions 63 and 64 provided on the one side Y1 and the other side Y2 in the second orthogonal direction Y is moved along the inner surface of the bone hole 110, and thus, straight movement in cutting in the vibration direction can be improved.

### (Sixth Embodiment)

The treatment system according to a sixth embodiment of the present invention that includes the ultrasound treatment instrument including the ultrasound probe will be described below. Note that, in the fifth embodiment, description of portions common to those in the first embodiment will be omitted as appropriate.

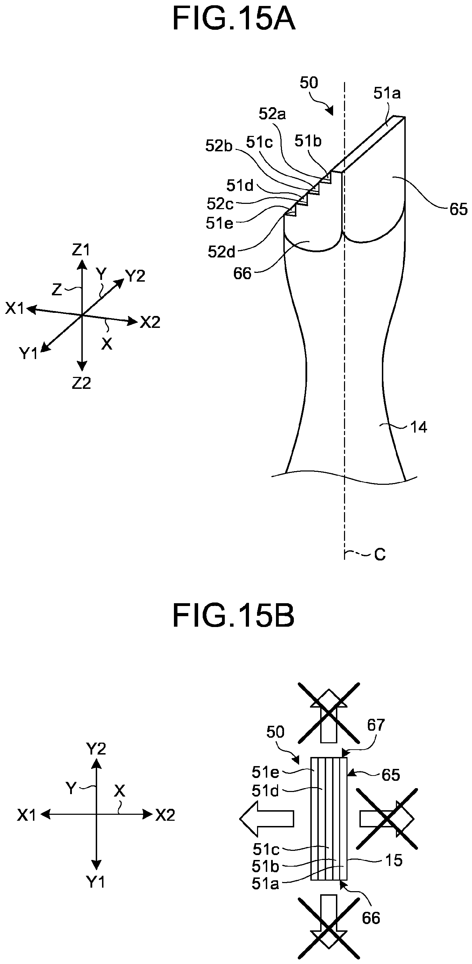

FIG. 15A is a perspective view illustrating the distal end side Z1 of the ultrasound probe 14 according to the sixth embodiment. FIG. 15B is a diagram of the distal end treatment portion 15 of the ultrasound probe 14 according to the sixth embodiment as viewed from the distal end side Z1 in the axial direction Z.

As illustrated in FIGS. 15A and 15B, in the distal end treatment portion 15 of the sixth embodiment includes, on both sides of the axis C, the striking face portion 50 that is provided on the one side X1 in the first orthogonal direction X and a side surface portion 65 that is provided on the other side X2 in the first orthogonal direction X. In addition, the distal end treatment portion 15 of the sixth embodiment includes side surface portions 66 and 67 formed into flat surfaces, on the one side Y1 and the other side Y2 in the second orthogonal direction Y, on both sides of the axis C.

In the striking face portion 50, a striking face 51a to a striking face 51e and a side surface 52a to a side surface 52d form a stair-like steps and a side surface connecting the first striking face and the second striking face that are arranged adjacently in the axial direction Z has a length (height of a step) that is equal to or less than the amplitude A of the ultrasound vibration. Therefore, the distal end treatment portion 15 ultrasonically vibrated with the amplitude A and being cutting the bone hole 110 is movable to the one side X1 in the first orthogonal direction X on which the striking face portion 50 is provided, as indicated by an arrow in FIG. 15B. On the other hand, the distal end treatment portion 15 ultrasonically vibrated with the amplitude A and being cutting the bone hole 110 cannot move to the other side X2 in the first orthogonal direction X on which the side surface portion 65 is provided and the one side Y1 and the other side Y2 in the second orthogonal direction Y on which the side surface portions 66 and 67 are provided, and the moving direction of the distal end treatment portion 15 is limited. This configuration makes it possible to suppress movement of the distal end treatment portion 15 ultrasonically vibrated with the amplitude A in an unintended direction during cutting in the bone hole 110.

In addition, in the distal end treatment portion 15 of the sixth embodiment, at least one of the side surface portion 65 provided on the other side in the first orthogonal direction X and the side surface portions 66 and 67 provided on the one side Y1 and the other side Y2 in the second orthogonal direction Y is moved along the inner surface of the bone hole 110, and thus, straight movement in cutting in the vibration direction can be improved.

### (Seventh Embodiment)

The treatment system according to a seventh embodiment of the present invention that includes the ultrasound treatment instrument including the ultrasound probe will be described below. Note that, in the seventh embodiment, description of portions common to those in the first embodiment will be omitted as appropriate.

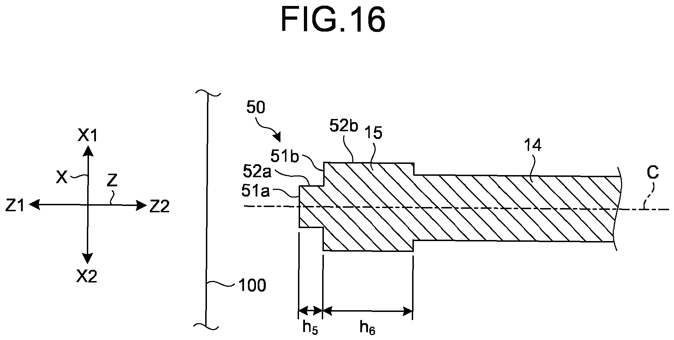

FIG. 16 is a cross-sectional view of the ultrasound probe 14 according to the seventh embodiment taken in the axial direction Z. Note that FIG. 16 illustrates the distal end side Z1 of the ultrasound probe 14.

The distal end treatment portion 15 of the seventh embodiment has a rectangular shape and includes a bottom surface that is connected to the probe body of the ultrasound probe 14.

The distal end treatment portion 15 according to the seventh embodiment includes the striking face portion 50 protruding toward the distal end side Z1 in the axial direction Z. The striking face portion 50 includes a striking face 51a as the first striking face and a striking face 51b as the second striking face that face the distal end side Z1 in the axial direction Z, and a side surface 52a as the first side surface and a side surface 52b as the second side surface that face in a direction orthogonal to the axial direction Z and each of which connects striking faces arranged adjacently in the axial direction Z.

The striking face 51a is a distal end surface of the distal end treatment portion 15 and is formed into a square flat surface. An angle between the side surface 52a and the striking face 51b is 90 degrees. Note that the angle between the side surface 52a and the striking face 51b may be smaller than 90 degrees.

The striking faces 51a and 51b and the side surfaces 52a and 52b form stair-like steps on the outer peripheral surface of the striking face portion 50.

In the distal end treatment portion 15 of the seventh embodiment, as illustrated in FIG. 16, a length h₆ of the side surface 52b in the axial direction Z is longer than a length h₅ of the side surface 52a in the axial direction Z. The length h₅ of the side surface 52a in the axial direction Z satisfies 0 < h₅ ≤ 200 [µm] and is equal to or less than the amplitude A of the ultrasound vibration. Meanwhile, the length h₆ of the side surface 52b in the axial direction Z satisfies h₆ > 200 [µm] and is larger than the amplitude A of the ultrasound vibration. Note that, in the distal end treatment portion 15 according to the embodiments described above, the step of the distal end treatment portion 15 is, for example, 200 [µm], whereas the distal end treatment portion 15 has a size of several [mm] or more. Therefore, the actual step of the distal end treatment portion 15 is visually very small.

Therefore, it is possible for the distal end treatment portion 15 ultrasonically vibrated to perform the vertical cutting and the horizontal cutting in the bone hole 110 without interruption due to the steps formed on the striking face portion 50, improving the straight movement upon vertical cutting by the side surface 52b.

The ultrasound treatment instrument that is a treatment cutting instrument used for orthopedic femur cutting, and treatment cutting thereof are described in the above embodiments, but the present invention is not limited to the above embodiments and is also naturally applicable to other cutting treatment instruments and other treatment cutting. In other words, when the present invention is applied to a treatment cutting instrument used for another clinical department, such as dentistry, and to treatment cutting of objects having various sizes, such as artificial bone and artificial tooth, the same effects as those described in the above embodiments are obtained.

### Industrial Applicability

As described above, the present invention can provide the ultrasound probe and the treatment system that are configured to perform the cutting in the vibration direction and the cutting in a direction orthogonal to the vibration direction without interruption.

### Reference Signs List

1 ULTRASOUND TREATMENT INSTRUMENT SYSTEM
2 ULTRASOUND TREATMENT INSTRUMENT
3 CONTROL DEVICE
4 FOOT SWITCH
10 TREATMENT SYSTEM
11 DEVICE BODY
11a HOUSING
11b ULTRASOUND TRANSDUCER UNIT
12 ULTRASOUND GENERATION UNIT
13 HORN
14 ULTRASOUND PROBE
15 DISTAL END TREATMENT PORTION
16 SHEATH
17 OPERATION SWITCH
18 FRONT SURFACE
19 CABLE
20 CONNECTORS
21 OPERATION SWITCH
22 DISPLAY SCREEN
30 ENDOSCOPE SYSTEM
31 ARTHROSCOPE
32 LIGHT SOURCE
33 CONTROL UNIT
34 INPUT UNIT
35 DISPLAY UNIT
36 FLUID SUPPLY/DISCHARGE UNIT
50 STRIKING FACE PORTION
100 TREATMENT TARGET REGION
110 BONE HOLE
150 DISTAL-END-SIDE STRIKING FACE PORTION
155 PROXIMAL-END-SIDE STRIKING FACE PORTION

## Claims

1. An ultrasound probe comprising
a treatment portion that is formed at a distal end of a transmission portion transmitting input vibration energy, the treatment portion being configured to vibrate with a predetermined amplitude to cut a treatment target object,
wherein the treatment portion includes:
a first striking face that faces the treatment target object;
a second striking face that faces the treatment target object at a position away from the first striking face toward a proximal end side in an axial direction; and
a first side surface that connects the first striking face and the second striking face in the axial direction and that has a predetermined length in the axial direction,
wherein when the predetermined length of the first side surface is h, 0 < h ≤ 200 [µm] is satisfied.

2. The ultrasound probe according to claim 1, wherein the predetermined length h of the first side surface satisfies 50 [µm] ≤ h ≤ 200 [µm].

3. The ultrasound probe according to claim 1, wherein the first side surface is recessed.

4. The ultrasound probe according to claim 1, wherein an angle between the first side surface and the second striking face is 90 degrees.

5. The ultrasound probe according to claim 1, wherein
the treatment portion includes
a second side surface that extends from an edge of the second striking face toward the proximal end side in the axial direction and that has a length different from the predetermined length of the first side surface in the axial direction.

6. The ultrasound probe according to claim 5, wherein the length of the second side surface in the axial direction is 200 [µm] or more.

7. A treatment system comprising:
the ultrasound probe according to claim 1; and
a control device configured to control the vibration energy supplied to the ultrasound probe,
wherein the control device includes:
a switching instruction portion configured to give an instruction to switch a drive mode; and
an energy supply portion configured to supply the vibration energy generating ultrasound vibration having different amplitudes according to the instruction from the switching instruction portion.

8. An ultrasound probe comprising
a treatment portion that is formed at a distal end of a transmission portion transmitting input vibration energy, the treatment portion being configured to vibrate with a predetermined amplitude to cut a treatment target object,
wherein the treatment portion includes:
a first striking face that faces the treatment target object;
a second striking face that faces the treatment target object at a position away from the first striking face toward a proximal end side in an axial direction; and
a first side surface that connects the first striking face and the second striking face in the axial direction and that has a predetermined length in the axial direction,
wherein the first side surface has a length that is equal to or less than an amplitude of vibration of the treatment portion caused by the input vibration energy.
